Europäisches Patentamt

European Patent Office

Office européen des brevets

(19)

(11) Publication number: **0 152 830**
A1

(12) # EUROPEAN PATENT APPLICATION

(21) Application number: 85100978.7

(22) Date of filing: 31.01.85

(51) Int. Cl.⁴: **C 12 N 15/00**
// C12R1:19

(30) Priority: 02.02.84 JP 18133/84

(71) Applicant: **THE GREEN CROSS CORPORATION, 15-1, Imabashi-1-chome Higashi-ku Osaka-shi, Osaka 541 (JP)**

(72) Inventor: **Nakagawa, Yukimitsu, 5-2-603, Hanazono-cho Kuzuha, Hirakata-shi Osaka (JP)**
Inventor: **Uno, Shusei, 1-24-9, Kitanakafuri, Hirakata-shi Osaka (JP)**
Inventor: **Nagai, Masanori, 10-10, Nishiyamawaki, Yahata-shi Kyoto (JP)**
Inventor: **Arimura, Hirofumi, 2-18-1-401, Uenosaka, Toyonaka-shi Osaka (JP)**

(43) Date of publication of application: 28.08.85
Bulletin 85/35

(84) Designated Contracting States: **BE DE FR GB NL SE**

(74) Representative: **von Kreisler, Alek, Dipl.-Chem. et al, Deichmannhaus am Hauptbahnhof, D-5000 Köln 1 (DE)**

(54) Vector.

(57) A novel vector wherein at least one promoter region is present between two different restriction enzyme cleavage sites capable of giving, upon cleavage, the same cohesive end.

The vector can serve as a basic form of multiple promoters. By constructing multiple promoters using this vector, more efficient protein synthesis by genetic engineering becomes possible.

EP 0 152 830 A1

0152830

VECTOR

BACKGROUND OF THE INVENTION

1. Field of the Invention

This invention relates to a novel vector.  More particularly, it relates to a vector characterized by at least one promoter region occurring therein between two restriction enzyme cleavage sites respectively recognized by two different restriction enzymes but giving upon cleavage the same cohesive end.

2. Description of the Prior Art

The promoter is a regulating factor which controls the genetic information to be borne by RNA.  It serves as a site to be recognized by RNA polymerase and as a site of binding of RNA polymerase and, as the RNA synthesis start point, plays a very important role in protein synthesis.

The lac promoter, trp promoter and $\beta$-lactamase promoter are promoters known to be markedly efficient in phenotypic expression in Escherichia coli as a host.

Studies in search of a more potent promoter were pursued and brought about hybrid promoters, for instance. The tac promoter composed of the trp promoter and lac promoter, for instance, is a well-known hybrid promoter.

As promoters for which yeasts serve as hosts, there may be mentioned the PGK promoter, ADH promoter and phoE promoter.

## SUMMARY OF THE INVENTION

In search of a more potent promoter, the present inventors conducted investigations into promoters each comprising a plurality of promoters connected in series.

As a result, they found that the provision of vectors containing a promoter region between two restriction enzyme cleavage sites respectively recognized by two different restriction enzymes but giving, upon cleavage, the same cohesive end makes it possible to construct vectors each containing a plurality of promoters in series and have now completed the present invention.

Thus, the present invention is concerned with vectors characterized by at least one promoter region occurring therein between two restriction enzyme cleavage sites respectively recognized by two different restriction enzymes but giving, upon cleavage, the same cohesive end.

## BRIEF DESCRIPTION OF THE DRAWINGS

In the accompanying drawings,

Fig. 1 shows pDR540 and pTL1 derived therefrom, the latter being a basic plasmid for multiple tac promoters;

Fig. 2 shows a scheme for constructing a multiple tac promoter from pTL1;

Fig. 3 shows a construction scheme for a multiple tac promoter;

Fig. 4 indicates restriction enzyme cleavage sites of pYN1;

Fig. 5 shows the DNA sequence of and around the trp promoter/operator region of pYN1;

Fig. 6 indicates restriction enzyme cleavage sites within and around the trp promoter/operator of pYN3;

Fig. 7 indicates restriction enzyme cleavage sites of pYN4;

Fig. 8 indicates restriction enzyme cleavage sites of pYN6;

Fig. 9 shows the DNA sequence in the trp promoter/ operator region of pYN6;

Fig. 10 indicates restriction enzyme cleavage sites of pYN8, pYN9, and pYN10;

Fig. 11 shows a scheme for the preparation of pYN20 to be used for preparing a multiple trp promoter by making use of the SalI and XhoI sites;

Fig. 12 shows a scheme for the preparation, from pYN20, of pYN21 which serves as a basic form of multiple trp promoters; and

Fig. 13 shows a scheme for the preparation of a double and a triple trp promoter by making use of the SalI and XhoI sites.

### DETAILED DESCRIPTION OF THE INVENTION

In accordance with the invention, "to have the same cohesive end" means that the portions rendered single-stranded upon restriction enzyme cleavage have the same base sequence. For instance, the BglⅡ recoginition
sequence is

<div align="center">

5' - A G A T C T - 3'

3' - T C T A G A - 5'

</div>

and, upon cleavage, 5' - G A T C - 3' becomes the cohesive
end. On the other hand, the BamHI recognition sequence is

<div align="center">

5' - G G A T C C - 3'

3' - C C T A G G - 5'

</div>

and, upon cleavage, 5' - G A T C - 3' becomes the cohesive
end. Other restriction enzyme combinations which give the
same cohesive end are SalI and XhoI, and BamHI and BclI.

The above fact makes possible the connection of both
ends (hereinafter referred to as "ligation"). This ligation sites is no more cleavable by the two enzymes used
previously due to the difference between both the enzyme
recognition sites. Taking advantage of this characteristic
property, it is possible to prepare multiple promoters.
For example, the BamHI and BglⅡ cohesive ends, when
ligated together, give the sequence

<div align="center">

5' - G G A T C T - 3'

3' - C C T A G A - 5'

</div>

and this site, which now the MboI cleavage site, cannot be
cleaved with BamHI or BglⅡ any more.

The SalI and XhoI cohesive ends, when ligated together,
give the sequence

<div align="center">

5' - G T C G A G - 3'

3' - C A G C T C - 5'.

</div>

0152830

This sequence is the TaqI cleavage site and therefore can no more be cleaved with SalI or XhoI.

In accordance with the invention, an operator region and an SD sequence can be present, together with the promoter region, between two different restriction enzyme recognition sites.

The operator region is a region which regulates the synthesis of mRNA corresponding to the adjacent structural gene through the interaction with a specific repressor, and the SD sequence is a ribosome binding site and is located in the neighborhood of and upstream from the protein synthesis initiation site.

The vector thus provided in accordance with the invention can serve as a basic form of multiple promoters. It is expected that by constructing multiple promoters using said vector, more efficient protein synthesis by genetic engineering becomes possible.

[Construction of multiple promoter]

The term "multiple promoter" as used herein means a plurality of repeatedly occurring, adjacent promoters. It may contain an operator and an SD sequence therein. When said operator and SD sequence are not contained in the promoter, the operator and SD sequence should be present in adjacency to and downstream from the multiple promoter.

Connecting a number of promoters in the above manner can lead to increased mRNA transcription efficiency.

The trp promoter is a promoter which enables efficient production of polypeptides and, when rendered multiple, can give a promoter capable of giving very high yields.

As one of the trp promoter, there may be mentioned a promoter having the sequence:

```
CCCTGTTGACAATTAATCATCGAACTAGTTAACTAGTACGCAAG
      ......           Taq I        .......
      -35                           -10
      Hinc II                       Hpa I   Rsa I

TTCACGTAAAAAGGGTATC
         ....
          SD
```

This promoter is prepared from pDR720 (available from PL Biochemical) and pGX112 (available from Genex). Although pDR720 has no SD sequence, the elements -35 region, -10 region and SD sequence, which are essential to the expression of genetic information, can be obtained by deriving therefrom a recombinant with pGX112. Said promoter has a size of about 70 bp and can easily be excised for use in heterologous gene expression.

Hybrid promoters can induce production of desired polypeptides in high yields and with high regulatability. For example, there may be mentioned the tac promoter which is a hybrid between the trp promoter and lac promoter, and the hybrid promoter between the rrn promoter and lac promoter.

The tac promoter comprises the trp ptomoter-derived -35 region (RNA polymerase recognition site) and the lac promoter-derived -10 region (RNA polymerase binding site, Pribnow box) and allows polypeptide production in high yields.

The tac promoter also has the lac promoter-derived operator and SD sequence, is controlled by the lactose repressor, and is advantageous in that it is very easily regulatable.

By making this tac promoter multiple, there can be obtained a highly regulatable promoter capable of affording very high polypeptide yields.

Such multiple tac promoter can be produced by deriving a basic vector, pTL1, from pDR540 (available from PL Biochemical).

A structural gene is inserted into the thus-obtained plasmid vector at a restriction enzyme cleavage site downstream from the promoter/operator (hereinafter abbreviated as "P/O") and a host is transformed with the insertion product, whereby the expression of said gene becomes possible. A preferred host is Escherichia coli.

Examples of the vector according to the invention are described below.

The DNA digestion and ligation are carried out by mixing necessary DNA (or DNAs) and enzyme (or enzymes) with an appropriate buffer for reaction (digestion or ligation) to prepare 50-100 μℓ of a reaction mixture having a final DNA concentration of about 1 μg/μℓ and

allowing the reaction to proceed for 2 hours to overnight. Enzymes are used in an amount of about 3 units per 1 µg of DNA. The reaction temperature is 12-16°C when T4 ligase is used, 50°C for BclI digestion, 30°C for SmaI digestion, 65°C for TaqI digestion, and 37°C for reactions with other enzymes.

The following are the compositions of reaction buffers each having a concentration 10 times that in carrying out the reaction. In using them in practice, they are added to the reaction system in amounts such that 10-fold dilution results.

| | |
|---|---|
| 10 x EcoRI buffer | 1.0M Tris-HCℓ, pH7.2 |
| | 50mM MgCℓ$_2$ |
| | 0.5M NaCℓ |
| 10 x HincII buffer | 60mM Tris-HCℓ, pH7.5 |
| | 60mM MgCℓ$_2$ |
| | 60mM β-mercaptoethanol |
| | 0.5M NaCℓ |
| 10 x HpaI buffer | 0.1M Tris-HCℓ, pH7.5 |
| | 0.1M MgCℓ$_2$ |
| | 10mM dithiothreitol |
| | 0.2M KCℓ |
| 10 x ClaI buffer | 60mM Tris-HCℓ, pH7.9 |
| | 60mM MgCℓ$_2$ |
| | 0.5M NaCℓ |

| | |
|---|---|
| 10 x <u>Pst</u>I buffer | 0.2M Tris-HCℓ, pH7.5 |
| | 0.1M MgCℓ$_2$ |
| | 0.5M (NH$_4$)$_2$SO$_4$ |
| 10 x <u>Rsa</u>I buffer | 60mM Tris-HCℓ, pH7.5 |
| | 0.12M MgCℓ$_2$ |
| | 60mM β-mercaptoethanol |
| | 0.5M NaCℓ |
| 10 x <u>Hae</u>II buffer | 0.1M Tris-HCℓ, pH7.5 |
| | 70mM MgCℓ$_2$ |
| | 70mM β-mercaptoethanol |
| 10 x <u>Hinf</u>I buffer | 0.06M Tris-HCℓ, pH7.4 |
| | 0.06M MgCℓ$_2$ |
| | 0.06M β-mercaptoethanol |
| | 0.5M NaCℓ |
| 10 x <u>Hind</u>III buffer | 0.06M Tris-HCℓ, pH7.4 |
| | 0.06M MgCℓ$_2$ |
| | 0.06M β-mercaptoethanol |
| | 0.5M NaCℓ |
| 10 x <u>Sal</u>I buffer | 0.1M Tris-HCℓ, pH7.5 |
| | 70mM MgCℓ$_2$ |
| | 70mM β-mercaptoethanol |
| | 2mM EDTA |
| | 1.75M NaCℓ |
| 10 x <u>Taq</u>I buffer | 0.1M Tris-HCℓ, pH7.5 |
| | 0.1M MgCℓ$_2$ |
| | 0.1M β-mercaptoethanol |
| | 1M NaCℓ |

| | |
|---|---|
| 10 x BglI buffer | 0.1M Tris-HCℓ, pH7.4 |
| | 0.1M MgCℓ$_2$ |
| | 10mM dithiothreitol |
| | 0.66M KCℓ |
| 10 x BglII buffer | 0.1M Tris-HCℓ, pH7.5 |
| | 70mM MgCℓ$_2$ |
| | 1M NaCℓ |
| | 70mM β-mercaptoethanol |
| 10 x AluI buffer | 60mM Tris-HCℓ, pH7.5 |
| | 60mM MgCℓ$_2$ |
| | 60mM β-mercaptoethanol |
| | 0.5M NaCℓ |
| 10 x AccI buffer | 60mM Tris-HCℓ, pH7.5 |
| | 60mM MgCℓ$_2$ |
| | 60mM β-mercaptoethanol |
| | 60mM NaCℓ |
| 10 x SmaI buffer | 0.1M Tris-HCℓ, pH8.0 |
| | 70mM MgCℓ$_2$ |
| | 70mM β-mercaptoethanol |
| | 0.2M KCℓ |
| 10 x BamHI buffer | 0.1M Tris-HCℓ, pH8.0 |
| | 70mM MgCℓ$_2$ |
| | 20mM β-mercaptoethanol |
| | 1M NaCℓ |

| | |
|---|---|
| 10 x <u>Xho</u>I buffer | 60mM Tris-HCℓ, pH7.9 |
| | 60mM MgCℓ$_2$ |
| | 60mM β-mercaptoethanol |
| | 1.5M NaCℓ |
| 10 x <u>Pvu</u>II buffer | 0.1M Tris-HCℓ, pH7.5 |
| | 70mM MgCℓ$_2$ |
| | 70mM β-mercaptoethanol |
| | 0.6M NaCℓ |
| T4 ligase buffer | 660mM Tris-HCℓ, pH7.6 |
| | 66mM MgCℓ$_2$ |
| | 100mM DTT |
| | 1-10mM ATP |

Example 1 (Preparation of multiple tac P/O)

The <u>Hind</u>III cleavage site of pDR540 was converted to the <u>Bgl</u>II cleavage site in the following manner.

Thus, pDR540 was digested with <u>Hind</u>III, followed by phenol/chloroform extraction and precipitation with ethanol. The precipitate obtained was dissolved in sterile water, heated at 65°C for 3 minutes and then used for the preparation of the following reaction mixture:

Reaction mixture:

| | |
|---|---|
| pDR540 <u>Hind</u>III cut | 150 μℓ |
| 10 x Nick translation buffer | 20 μℓ |
| 2mM each of dNTP (four kinds) | 10 μℓ each |
| DNA polymerase large fragment | 20 μℓ (15 units) |

Composition of 10 x Nick translation buffer:

0.5M Tris-HCℓ, pH7.2

0.1M MgSO$_4$

1mM dithiothreitol

The above reaction mixture was kept at room temperature (28-30°C) for 40 minutes. To the reaction mixture obtained were added 100 μℓ of water-saturated redistilled phenol and 100 μℓ of chloroform. After 30 seconds of vigorous vortexing, the mixture was centrifuged for 2 minutes. The aqueous phase (200 μℓ) was taken and subjected to precipitation with ethanol. The precipitate was washed with 70% ethanol and dried. The dried precipitate was dissolved in 38 μℓ of water, then 5 μg (5 μℓ) of BglⅡ linker [Takara Shuzo's phosphorylated BglⅡ linker d(pCAGATCTG)] was added, and the mixture was heated at 65°C for 3 minutes and cooled at room temperature.

Reaction mixture:

| | |
|---|---|
| Above DNA mixture | 43 μℓ |
| 10 x T4 ligase buffer | 5 μℓ |
| T4 ligase | 2 μℓ (5.6 units) |

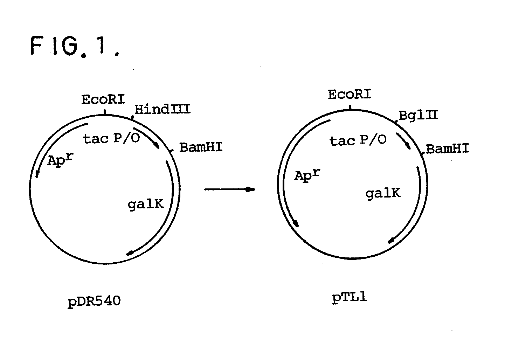

The above reaction mixture was kept at 16°C overnight to effect ligation of the BglⅡ linker. The DNA obtained was digested with BglⅡ, the resultant reaction mixture was electrophoresed on 1% agarose, and a DNA band corresponding to about 4,000 bp was recovered by electroelution [G. Dretzen, H. Bellard, P. Sassone-Corsi, and P. Chambon (1981), Anal. Biochem., 112, 295-298]. The DNA fragment thus obtained was ligated by the conventional method. The plasmid obtained was used for transformation of E. coli JM103. Transformant selection was performed making advantage of ampicillin resistance, and DNA was extracted therefrom. The DNA obtained was subjected to BglII digestion, HindIII digestion, EcoRI-BglII double digestion (hereinafer, "EcoRI + BglII digestion"), BglII + EcoRI digestion, and BamHI + EcoRI digestion, followed by 5% polyacrylamide gel electrophoresis, which confirmed formation of a pDR540-derived plasmid containing a BglII cleavage site in lieu of the original HindIII cleavage site. Said plasmid was named pTL1 (Fig. 1).

The thus-obtained pTL1 had BglII and BamHI as two different restriction enzyme cleavage sites and had a tac P/O between them.

This pTL1 was then digested with EcoRI + BamHI to give a tac P/O-containing fragment having a size of about 380 bp. Separately, pTL1 was digested with EcoRI + BglII to give a fragment about 4 kb in size. These two fragments were ligated together, and E. coli JM103 was transformed with the ligation product, followed by transformant selection by means of ampicillin resistance. More than 10,000 transformants were obtained. From 47 of them, plasmid DNA was extracted by the miniprep method [H. C. Birnboim and J. Doly (1979), Nucl. Acids Res., 7, 1513] and digested with BamHI + BglII , whereby a fragment about 200 bp in size was obtained from 45 out of the 47 transformants.  Plasmid DNA was extracted in large amounts from
such transformants and digested with BamHI + BglII , EcoRI +
BamHI, and EcoRI + BglII .  The results of the digestions
are shown in Table 1.  These plasmids each is a plasmid
having a double tac P/O, which plasmid was named pTL2.

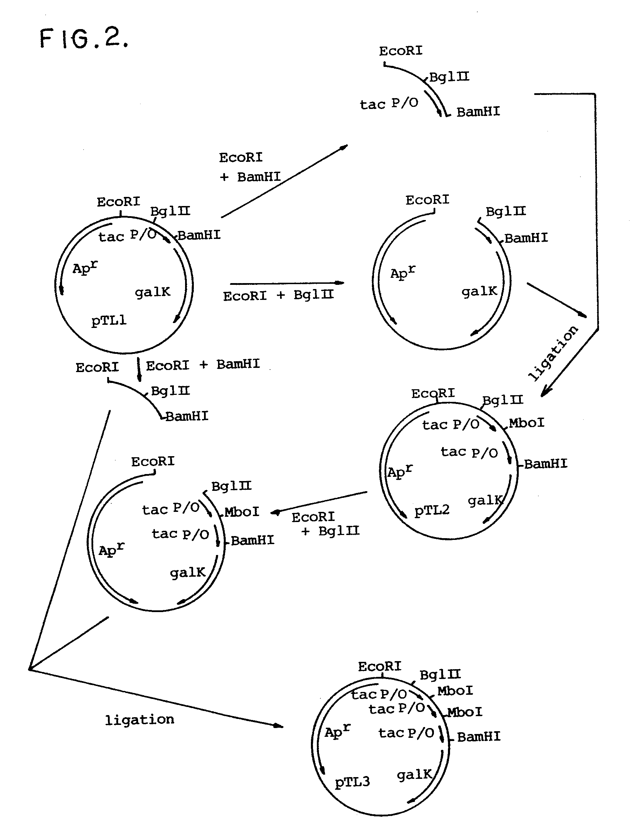

Then, the 380 bp fragment as obtained by EcoRI +
BamHI digestion of pTL1 and the 4 kb fragment as obtained
by EcoRI + BglII digestion of pTL2 were ligated together
and the ligation product was used for transformation of
E. coli JM103.  Selection by means of ampicillin resistance
gave more than 10,000 transformants, of which 30 were
subjected to plasmid DNA extraction by the miniprep method,
followed by BamHI + BglII digestion.  The plasmid DNA
from 29 transformants gave a fragment about 300 bp in size.
These plasmids were prepared in large amounts and subjected
to the same restriction enzyme digestions as applied to
pTL2.  The results obtained are shown in Table 1.  These
plasmids each is a plasmid having a triple tac P/O.  The
plasmid was named pTL3 (Fig. 2).

Table 1

|  | pTL1 | pTL2 | pTL3 |
|---|---|---|---|
| BamHI + BglII digestion | 98 bp | 195 bp | 295 bp |
| EcoRI + BamHI digestion | 400 bp | 495 bp | 600 bp |
| EcoRI + BglII digestion | 305 bp | 305 bp | 300 bp |

- 15 -                            0152830

Example 2 (Preparation of multiple trp P/O)

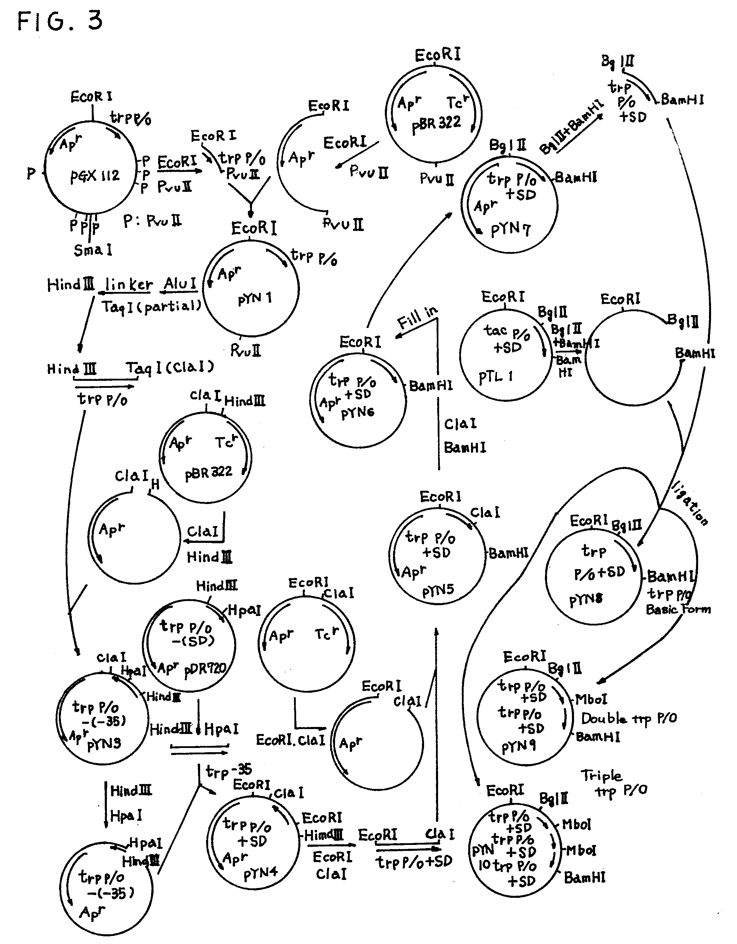

[Hereinafter pYN1 to pYN7 are precursor plasmids.]
[The scheme for preparation is outlined in Fig. 3.]
(1) (Preparation of pYN1)

pGX 112 was subjected to EcoRI + PvuII + PstI digestion, which was performed in the conventional manner,
followed by 1% agarose electrophoresis.  A band corresponding to 2.95 kb (a band in which a trp promoter region
was present) was recovered by the method of Yang et al.
[R. C. A. Yang, L. Lis, and R. Wu (1979), Methods in
Enzymology, 68, 176-182].  pBR322 was likewise digested
with EcoRI + PvuII + SalI and a 2.25 kb band was reovered
by electroelution.

Then, the two DNA fragments obtained were ligated
together by the conventional method.  After ligation, E.
coli RRl was transformed with the product.  From the transformants obtained, plasmid DNA was extracted by the miniprep
method.  The DNA (as it was in the cccDNA form) was electrophoresed on 1% agarose, and a 5.2 kb plasmid expected
from calculation to be the desired one was obtained (from
1 out of 20 transformants).

This plasmid DNA was digested with the restriction
enzyme HpaI, followed by 5% polyacrylamide gel electrophoresis.  As a result, 230 and 320 bp fragments characteristic of the base sequence in the neighborhood of trp
P/O were obtained.  The plasmid was named pYN1.

For pYN1, a restriction enzyme map was prepared.

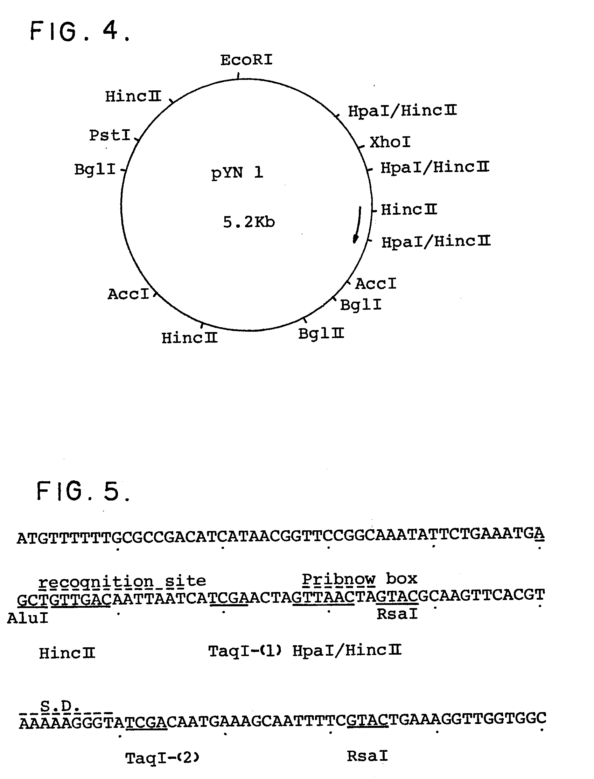

Fig. 4 is a partial representation of said map.  This pYN1
was examined for DNA sequence around the trp P/O region by
the Maxam-Gilbert method (Fig. 5).  The base sequence each
of the -35 region, -10 region and SD sequence was in agreement with the data in the following references:

    oJ. D. Windass, C. R. Newton, J. De Maeyer-Guignard, V. E. Moore, A. F. Markham, and M. D. Edge (1982) Nucl. Acids Res., 10, 6639-6657

    oF. Lee, K. Bertrand, G. Bennett, and C. Yanofsky (1978) J. Mol. Biol., 121, 193-217

(2) (Preparation of pYN3)

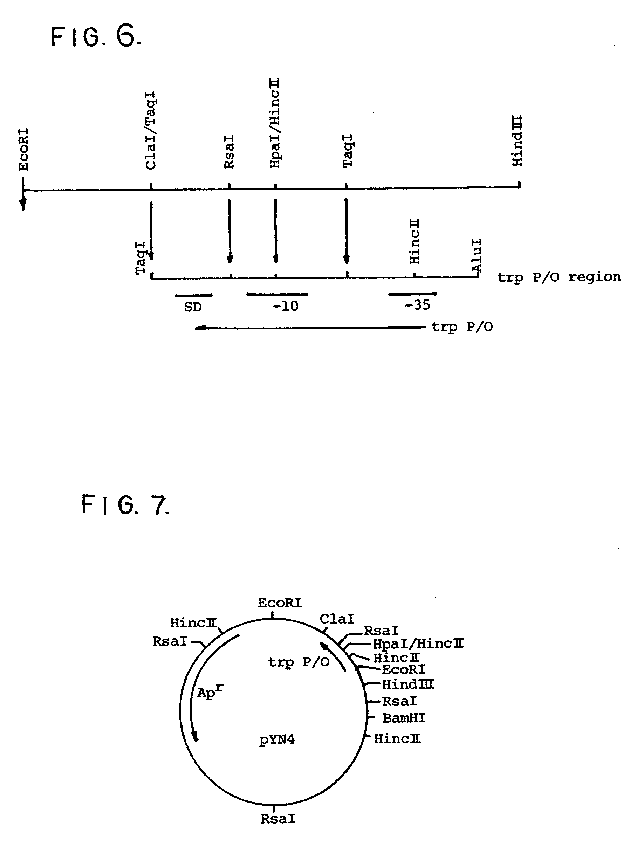

For the purpose of preparing a more compact trp P/O,
the portion from the AluI site to the TaqI-(2) site as
shown in Fig. 5 was isolated.  Thus, pYN1 was first di-
gested with AluI to give a trp P/O region-containing
fragment about 340 bp in size (Fig. 6).  To each end of
this fragment was joined a HindIII linker [Takara Shuzo's
phosphorylated HindIII linker d(pCAAGCTTG)] by overnight
reaction at 16°C in the reaction mixture given below.

Reaction mixture:

| | |
|---|---|
| 340 bp AluI fragment | 30 µℓ |
| HindIII linker | 2 µg (2 µℓ) |
| 10 x T4 ligase buffer | 5 µℓ |
| T4 ligase | 5 units (4 µℓ) |
| $H_2O$ | 10 µℓ |

0152830

To 50 μℓ of the resultant HindⅢ digestion reaction mixture, there was added 18 units of TaqI and partial digestion was conducted at 65°C for 5 minutes, followed by phenol/chloroform extraction, ethanol precipitation, and dissolution in sterile water.

The thus-obtained HindⅢ-TaqI fragment of pYN1 was inserted into pBR322 at the ClaI-HindⅢ site.

Then, E. coli RR1 and E. coli HB101 were transformed with the plasmid obtained. Transformant selection was performed taking advantage of ampicillin resistance, giving more than 10,000 E. coli RR1 transformants and about 300 E. coli HB101 transformants. Plasmid DNA was extracted from 120 transformants by the miniprep method, cleaved with HpaI, electrophoresed on 1% agarose and examined for the presence of a HpaI site within the trp P/O region. As a result, two clones were estimated to have such HpaI site and therefore digested with various restriction enzymes and then electrophoresed. The map of restriction enzyme cleavage sites in the neighborhood of the trp P/O as expected from the cleavage pattern found is shown in Fig. 6. These plasmid DNAs were lacking in HincⅡ site which must be present in the -35 region, but the -10 region and SD sequence were actually present therein. These plasmids were named pYN3.

(3) (Preparation of pYN4)

It was decided that the SD sequence-deficient trp

P/O of pDR720 and the SD sequence of pYN3 be connected at the HpaI site.

Thus, pDR720 was digested with HpaI + HindIII, followed by electrophoresis on 5% polyacrylamide gel, whereby a band corresponding to about 60 bp was eluted. Separately, pYN3 was digested with HpaI + HindIII and then treated with bacterial alkaline phosphatase (hereinafter, "BAP")(BRL).

The HpaI-HindIII fragment (about 60 bp) from pDR720 and the HpaI-HindIII fragment (about 4 kb) from pYN3 were ligated together, and the ligation product was used for transformation of E. coli HB101. Selection by means of ampicillin resistance gave 13 transformants. DNA was extracted from these transformants by the miniprep method and subjected to HindIII + ClaI digestion and HindIII + ClaI + HpaI digestion. With all clones, a fragment about 58 bp in size and a fragment about 32 bp in size were obtained. Based on this fact, it was considered that the desired plasmid was in hand. This plasmid was extracted in large amounts and, based on cleavage patterns obtained by digesting it with various restriction enzymes, its restriction enzyme cleavage map was prepared (Fig. 7). The plasmid was named pYN4.

(4) (Preparation of pYN5)

Then, the following experiment was carried out to make this trp P/O region present between BglII and BamHI sites.

pYN4 was digested with EcoRI + ClaI to give a fragment about 66 bp in size. Separately, pBR322 was digested with EcoRI + ClaI and then treated with BAP, giving a fragment about 4 kb in size. These two fragments were ligated together, and E. coli HB101 was transformed with the ligation product. Selection by means of ampicillin resistance gave 22 transformants, from each of which DNA was extracted by the miniprep method and digested with EcoRI + ClaI. All clones gave a 60-70 bp fragment. One of the clones was named pYN5.

(5) (Preparation of pYN6)

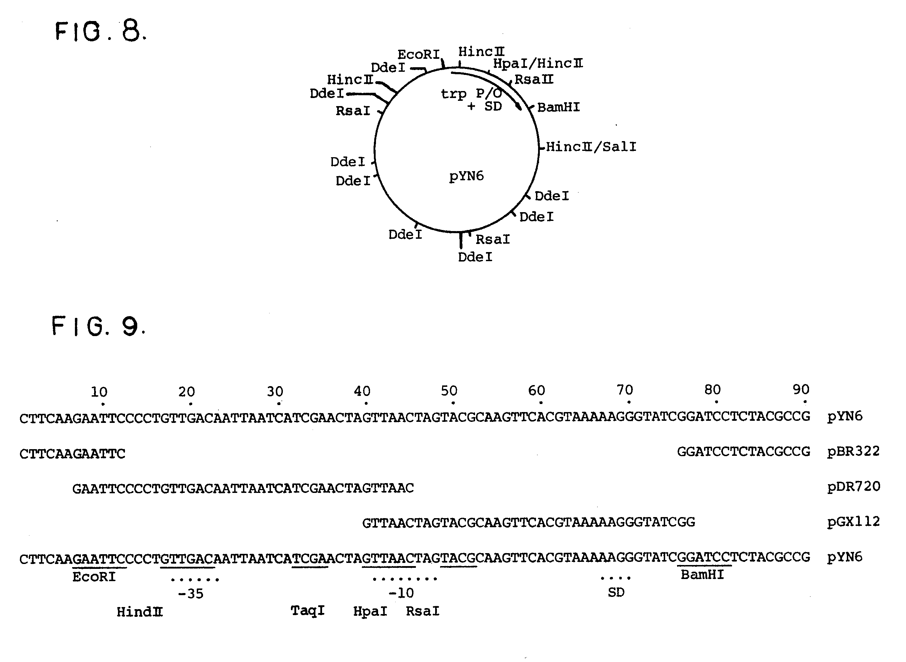

To convert the ClaI site of pYN5 to a BamHI site, pYN5 was digested with BamHI + ClaI. The single-stranded portion at each site was converted to a complementary double-strand using DNA polymerase I Klenow fragment (NEB) to give a fragment about 4 kb in size. This fragment was subjected to ligation and the product was used for transforming E. coli HB101. Selection by means of ampicillin resistance gave 27 transformants. The DNA plasmid from 14 out of these was cleavable with BamHI but not cleavable with ClaI. Therefore, it was concluded that said plasmid was the desired one, and its restriction enzyme cleavage map was examined (Fig. 8). (The plasmid was named pYN6.) Furthermore, the base sequence of the trp P/O region of pYN6 was studied by the Maxam-Gilbert method. It was revealed that the trp P/O region of pYN6 contained the -35 region, -10 region and SD sequence. Upon EcoRI + BamHI digestion, said region could be excised as a fragment having a size of about 70 bp (Fig. 9).

(6) (Preparation of pYN7)

In the next place, an attempt was made to convert the EcoRI site of pYN6 to a BglII site. Thus, pYN6 was treated with EcoRI and then with SI nuclease, and the single-stranded portion was rendered double-stranded using DNA polymerase I Klenow fragment. Thereafter, in the same manner as above, a BglII linker was joined thereto, followed by treatment with BglII and ligation. E. coli HB101 was transformed with the ligation product. Selection by means of ampicillin resistance gave more than 10,000 transformants. Of 40 of said transformants, one was cleavable with BglII but uncleavable with EcoRI and found to be the desired plasmid. (It was named pYN7.)

(7) (Preparation of pYN8, pYN9 and pYN10)

pYN7 was digested with BglII + BamHI to give a fragment about 70 bp in size. Separately, pTL1 of Example 1 was subjected to BglII + BamHI digestion and treatment with BAP, which gave a fragment about 4 kb in size. These two fragments were ligated together, and the ligation product was used for transforming E. coli HB101. By selection using ampicillin resistance, more than 10,000 transformants were obtained. Out of 54 transformants investigated, 13 gave plasmid DNA cleavable with both BglII and BamHI. The plasmid DNA was further subjected to BglII + BamHI digestion, and BglII + BamHI + HpaI digestion.  As a result, the plasmid DNA from 8 clones (named pYN8), upon BglII + BamHI digestion, gave a fragment about 75 bp in size.  The BglII + BamHI + HpaI digestion led to disappearance of the band corresponding to about 75 bp.  The plasmid DNA from other 3 clones (named pYN9) gave, upon BglII + BamHI digestion, a fragment about 165 bp in size and, upon BglII + BamHI + HpaI digestion, a fragment about 75 bp in size.  The plasmid DNA for a further clone (named pYN10) gave, upon BglII + BamHI digestion, a fragment about 240 bp in size and, upon BglII + BamHI + HpaI digestion, a fragment about 75 bp in size.

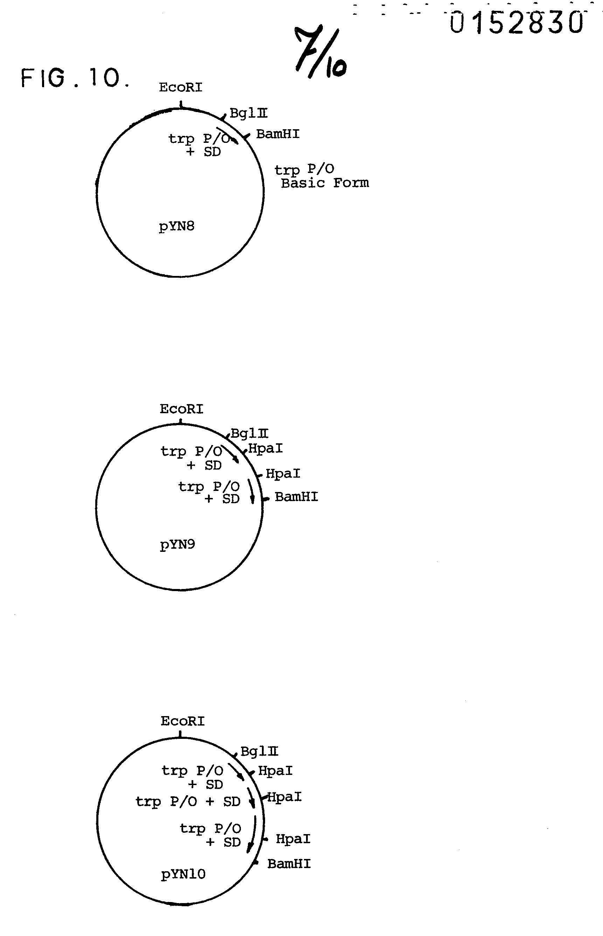

Each clone was cultured on a large scale and plasmid DNA was prepared by the cleared lysate method.  The plasmid DNA was digested with various restriction enzymes, followed by electrophoresis.  The size of each fragment obtained was determined.  The restriction enzyme cleavage maps obtained in this manner are shown in Fig. 10.  pYN8 was a plasmid serving as a basic form of multiple trp P/O, whereas pYN9 and pYN10 were double trp P/O- and triple trp P/O-containing plasmids, respectively.

Example 3 (Preparation of multiple trp promoter)

(1) (Preparation of pYN20)

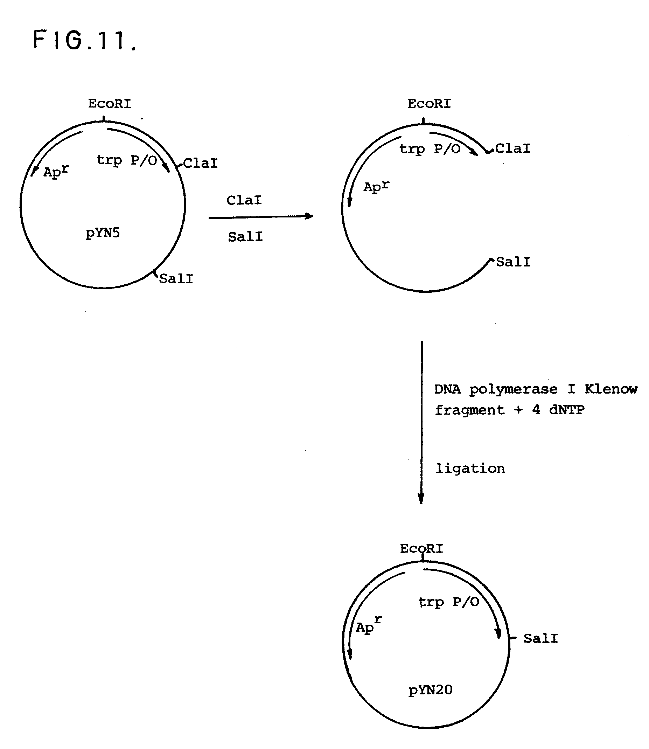

The procedure was as outlined in Fig. 11.  The plasmid pYN5 prepared in Example 2 was subjected to ClaI + SalI digestion, followed by 1% agarose electrophoresis and electroelution, which gave a fragment about 3.8 kb in size.

Its cohesive ends were filled in using DNA polymerase I Klenow fragment in the presence of the four dNTPs. The subsequent ligation using T4 DNA ligase led to disappearance of the ClaI site and regeneration of a SalI site. Using the resultant DNA, E. coli HB101 was transformed. Selection by means of ampicillin resistance gave more than 10,000 transformants, from 24 of which plasmid DNA was extracted by the miniprep method. The DNA was divided into two portions. One portion was digested with SalI and the other with ClaI, followed by 1% agarose electrophoresis in each case. The plasmid from 13 transformants was cleavable with SalI but uncleavable with ClaI and therefore was the desired plasmid, and was named pYN20.

(2) (Preparation of pYN21)

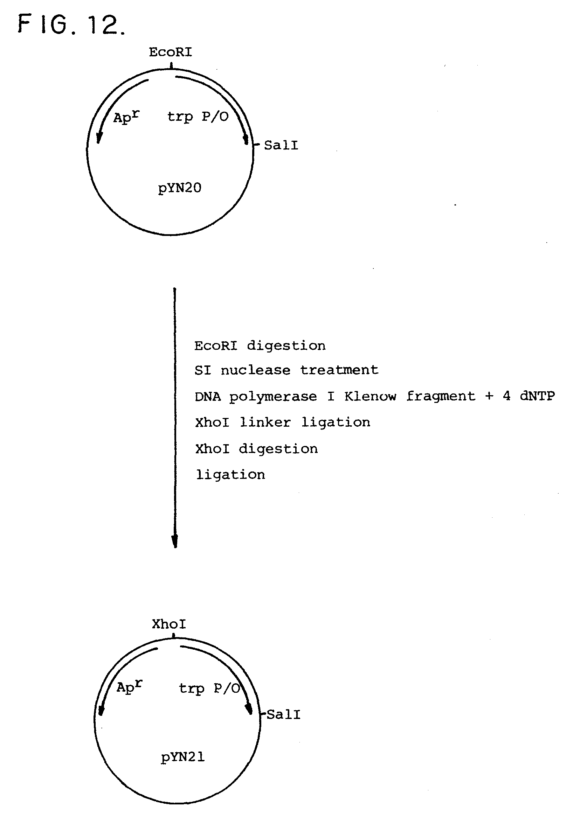

For converting the EcoRI site of pYN20 to a XhoI site, the procedure outlined in Fig. 12 was followed. Thus, pYN20 was digested with EcoRI, followed by phenol/chloroform extraction and precipitation with ethanol. The DNA thus obtained was treated with SI nuclease for cohesive end digestion. Three phenol/chloroform extractions followed by ethanol precipitation gave a precipitate, which was dissolved in sterile water, heated at 65°C for 3 minutes and then cooled rapidly. Thereafter, treatment with DNA polymerase I Klenow fragment was conducted in the presence of 4 dNTPs, followed by phenol/chloroform extraction and ethanol precipitation, and further by ligation of a XhoI linker [Takara Shuzo's XhoI linker d(CCTCGAGG)]. After the subsequent digestion with XhoI, the digest was electrophoresed on 1% agarose and a fragment about 3.8 kb in size was electroeluted. This fragment was ligated using T4 DNA ligase and the ligation product was used for transforming E. coli HB101. Selection through ampicillin resistance gave more than 10,000 transformants, 24 of which were subjected to plasmid DNA extraction. The DNA was divided into two portions. One portion was digested with XhoI and the other with EcoRI. The DNA from 3 transformants was not digestible with EcoRI but was digestible with XhoI and found to be the desired one. This was named pYN21.

(3) (Preparation of multiple trp promoter)

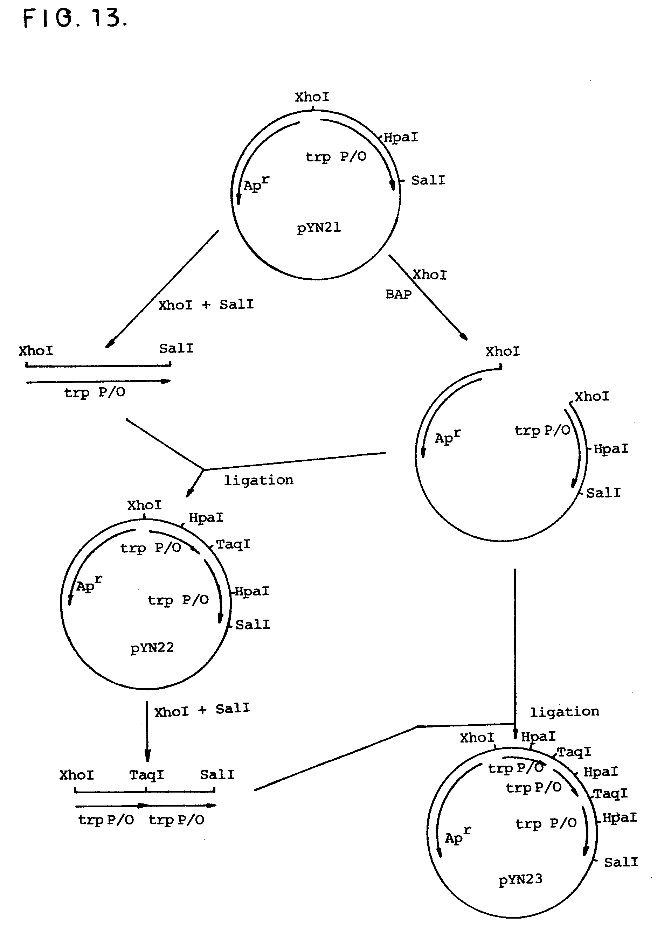

The procedure followed was as outlined in Fig. 13. Thus, pYN21 was digested with XhoI + SalI. The subsequent 5% polyacrylamide electrophoresis gave a fragment about 75 bp in size. Separately, pYN21 was digested with XhoI and then treated with BAP. Three repetitions of phenol/ chloroform extraction and the subsequent ethanol precipitation gave a precipitate, which was ligated with the 75 bp fragment. E. coli HB101 was transformed with the ligation product. Upon selection by means of ampicillin resistance, more than 10,000 transformants were obtained. From 20 of them, plasmid DNA was extracted and digested with XhoI + SalI and with HpaI. Each digest was treated with RNase and electrophoresed on 5% polyacrylamide gel.

The DNA from 4 transformants gave a fragment about 160 bp in size upon XhoI + SalI digestion and a fragment about 75 bp in size upon HpaI digestion.  This plasmid was estimated to contain a double trp promoter, and named pYN22. pYN22 was then prepared in large amount, digested with XhoI + SalI and electrophoresed on 5% polyacrylamide gel. A fragment about 160 bp in size was thus obtained.  This fragment was ligated with the XhoI fragment from pYN21 (mentioned hereinabove) and the product was used for transforming E. coli HB101.  Selection by means of ampicillin resistance gave about 1,000 transformants.  From 30 of them, plasmid DNA was extracted and digested with XhoI + SalI and with HpaI.  The plasmid DNA from one transformant gave a fragment about 230 bp in size upon XhoI + SalI digestion and a fragment about 75 bp in size upon HpaI digestion.  This was the desired plasmid containing a triple trp P/O, and was named pYN23.

0152830

What we claim are:

1. A vector characterized by at least one promoter region occurring therein between two restriction enzyme cleavage sites respectively recognized by two different restriction enzymes but giving upon cleavage the same cohesive end.

2. The vector of Claim 1, wherein said two different restriction enzymes are BglII and BamHI.

3. The vector of Claim 1 or 2, characterized by an EcoRI cleavage site occurring therein upstream from the BglII and BamHI cleavage sites.

4. The vector of Claim 1, wherein said two different restriction enzymes are SalI and XhoI.

5. The vector of Claim 1 or 4, characterized by an EcoRI cleavage site occurring therein upstream from the SalI and XhoI cleavage sites.

6. The vector of Claim 1, wherein an operator region and an SD (Shine-Dalgarno) sequence are contained therein between two or more different cleavage sites.

7. The vector of Claim 1, which is a plasmid.

8. The vector of Claim 1, wherein a strain of Escherichia coli serves as the host to said vector.

9. The vector of Claim 1, wherein said promoter is a trp promoter.

10. The vector of Claim 9, wherein said trp promoter has the following base sequence:

```
CCCTGTTGACAATTAATCATCGAACTAGTTAACTAGTACGCAAG
     ......           ......
     -35         TaqI        -10
     HincII                HpaI    RsaI

TTCACGTAAAAAGGGTATC
         ....
          SD
```

11.    The vector of Claim 1, wherein said promoter is a hybrid promoter.

12.    The vector of Claim 11, wherein said hybrid promoter is a hybrid between a trp promoter and a lac promoter.

13.    The vector of Claim 1, characterized by a plurality of promoters occurring therein in series.

14.    The vector of Claim 1, which contains a structural gene inserted therein at that cleavage sites out of the two restriction enzyme cleavage sites with the promoter therebetween which is downstream from the other.

15.    The vector of Claim 1, wherein the basic vector is a plasmid derived from the plasmid pDR540.

16.    The vector of Claim 1, wherein the basic vector is a plasmid derived from the plasmids pDR720 and pGX112.

1/10

FIG. 1.

pDR540 → pTL1

FIG.2.

2/10

FIG. 3

# FIG. 4.

EcoRI

HincII

PstI

Bg1I

pYN 1

5.2Kb

HpaI/HincII

XhoI

HpaI/HincII

HincII

HpaI/HincII

AccI

BglI

AccI

HincII

BglII

# FIG. 5.

ATGTTTTTTGCGCCGACATCATAACGGTTCCGGCAAATATTCTGAAATGA

recognition site                   Pribnow box
GCTGTTGACAATTAATCATCGAACTAGTTAACTAGTACGCAAGTTCACGT
AluI                                          RsaI

    HincII              TaqI-(1) HpaI/HincII

    S.D.
AAAAAGGGTATCGACAATGAAAGCAATTTTCGTACTGAAAGGTTGGTGGC

    TaqI-(2)                      RsaI

# FIG. 6.

# FIG. 7.

## FIG. 8.

## FIG. 9.

```
        10        20        30        40        50        60        70        80        90
         •         •         •         •         •         •         •         •         •
CTTCAAGAATTCCCCTGTTGACAATTAATCATCGAACTAGTTAACTAGTACGCAAGTTCACGTAAAAAGGGTATCGGATCCTCTACGCCG   pYN6

CTTCAAGAATTC                                                                GGATCCTCTACGCCG   pBR322

    GAATTCCCCTGTTGACAATTAATCATCGAACTAGTTAAC                                                  pDR720

                             GTTAACTAGTACGCAAGTTCACGTAAAAAGGGTATCGG                           pGX112

CTTCAAGAATTCCCCTGTTGACAATTAATCATCGAACTAGTTAACTAGTACGCAAGTTCACGTAAAAAGGGTATCGGATCCTCTACGCCG   pYN6
    EcoRI     ......                    ........                     ....     BamHI
                -35                      -10                          SD
       HindⅡ                    TaqI   HpaI  RsaI
```

FIG.10.

FIG.11.

FIG. 12.

EcoRI

Ap$^r$    trp P/O

SalI

pYN20

EcoRI digestion

SI nuclease treatment

DNA polymerase I Klenow fragment + 4 dNTP

XhoI linker ligation

XhoI digestion

ligation

XhoI

Ap$^r$    trp P/O

SalI

pYN21

FIG. 13.

0152830

Application number

European Patent
Office

**EUROPEAN SEARCH REPORT**

| | **DOCUMENTS CONSIDERED TO BE RELEVANT** | | EP 85100978.7 |

| Category | Citation of document with indication, where appropriate, of relevant passages | Relevant to claim | CLASSIFICATION OF THE APPLICATION (Int. Cl.4) |
|---|---|---|---|
| A | EP - A2 - 0 092 388 (ELI LILLY AND COMPANY)<br>* Fig. 1,2 * | 1,2 | C 12 N 15/00<br>// C 12 R 1:19 |
| A | EP - A2 - 0 068 740 (ELI LILLY AND COMPANY)<br>* Fig. 3,4 * | 1,2 | |

TECHNICAL FIELDS
SEARCHED (Int. Cl.4)

C 12 N

The present search report has been drawn up for all claims

| Place of search | Date of completion of the search | Examiner |
|---|---|---|
| VIENNA | 03-05-1985 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82